# EUROPEAN PATENT SPECIFICATION

(11) **EP 0 967 914 B1**
(45) Date of publication and mention of the grant of the patent: **28.07.2004**
(21) Application number: 98919094.7
(22) Date of filing: 12.03.1998
(51) Int. Cl.: A61B 5/04, A61B 5/0428

(54) **METHOD FOR BODY SURFACE MAPPING**
VERFAHREN ZUR DARSTELLUNG DER SIGNALE VON EINER KÖRPEROBERFLÄCHE
METHODE POUR LA CARTOGRAPHIE DE SURFACES CORPORELLES

(30) Priority: 12.03.1997 IE 970182; 12.03.1997 IE 970183; 12.03.1997 US 40440 P
(43) Date of publication of application: 05.01.2000
(73) Proprietor: Anderson, John McCune, Holywood County Down BT18 0NG (GB); Allen, James, Ballyclare County Antrim BT39 0BS (GB)
(72) Inventor: Anderson, John McCune, Holywood County Down BT18 0NG (GB); Allen, James, Ballyclare County Antrim BT39 0BS (GB)
(74) Representative: Casey, Lindsay Joseph
(86) International application number: PCT/EP1998/001446
(87) International publication number: WO 1998/040010

(56) References cited:
- US-A- 5 054 496
- US-A- 5 419 337
- J.J. SCHMID ET AL.: "Cardiogoniometry, a new technique for detecting ischemic heart disease" IEEE COMPUTERS IN CARDIOLOGY, 7 - 10 October 1986, BOSTON (US), pages 313-316, XP000041536

## Description

### Background of The Invention

This invention relates to a method of body surface mapping, in particular for use in diagnosing different conditions of the human myocardium. There are various methods which present the information contained in an ECG (ElectroCardioGraph) to the cardiologist, the most successful of which has been the standard twelve lead ECG. Unfortunately the standard ECG, in many instances, fails to provide an unequivocal diagnosis. One particular complication which renders the twelve lead ECG virtually useless involves the diagnosis of acute MI (Myocardial Infarction) in the presence of LBBB (Left Bundle Branch Block). Even before the conventional twelve lead ECG became a medical standard, electrocardiographic Body Surface Mapping (BSM) was being investigated as a method which increased spatial resolution and thereby increased diagnostic capability.

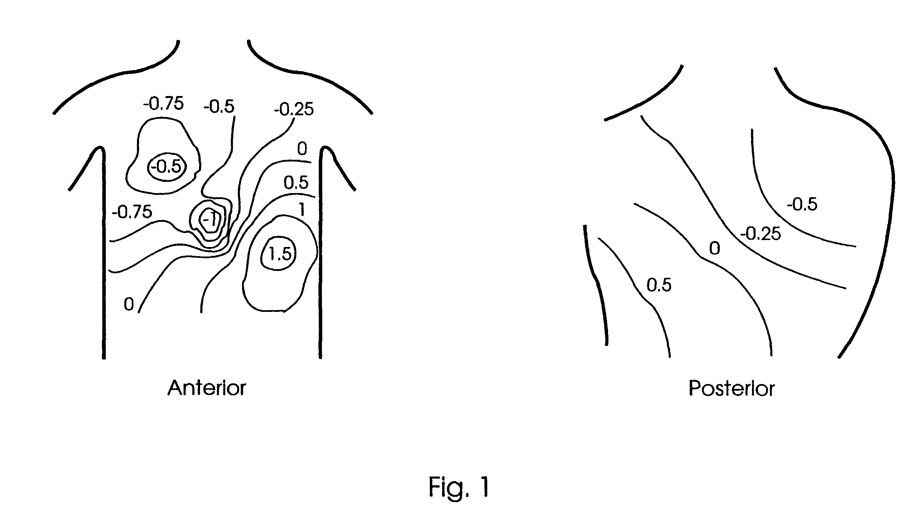

These "maps" were viewed as pictures presenting lines joining areas of the same electrical potential (isopotentials) at a specified instant of time. This is shown in Fig. 1, which is an isopotential map of a normal healthy subject mid-way through the heart's QRS (ventricular depolarisation) period. In Fig. 1 the map has been superimposed onto the outline of a human torso and is obtained from multiple ECG electrodes located substantially fully around the torso from the anterior (left hand side of Fig. 1) to the posterior (right hand side of Fig. 1). Since each map shows the isopotentials at only a single point in time, to see the whole electrical "picture" requires the viewing of successive maps at successive time instants throughout at least part of the cardiac cycle. In this manner however, BSM is difficult to use and as a result much research has been carried out which attempts to bring the benefits of BSM in a fast, easy to use form.

Methods have been introduced of portraying BSM information as mathematical integrations of the ECG waveforms which are recorded from each individual electrode site. The idea of integrating an ECG signal over a pre-defined interval was first conceived by Wilson and is described in detail in his publication FN Wilson et al. "The Determination and the Significance of the Areas of the Ventricular Deflections of the Electrocardiogram", The American Heart Journal, Vol 10, pp46-61, 1934. Using this mathematical integration over all ECG signals recorded over the body surface allows maps to be constructed which present the body surface as lines which join areas possessing the same integral values. The integrations are performed over pre-defined time intervals of the ECG. Such isointegral maps have shown that there is more information outside the spatial scope of the standard twelve lead ECG, which could be used by a clinician to improve patient management. These isointegral maps have proved their ability to provide an accurate diagnosis in acute instances where the standard twelve lead ECG was equivocal. This information however, is presented as a pattern, either in the form of contours, colours or three dimensional graphical representations. A knowledge based diagnosis must then be made by comparing the patterns obtained from any presented patient, to those previously experienced from other patients with known conditions.

The need to automate and increase the speed of diagnosis, resulted in the use of discriminant type statistical functions which can receive as input the integral values from the acquisition equipment and produce a statistical index of the likelihood that any given subject belongs to a pre-defined set of diagnostic groups.

The mathematical nature of these isointegrals provides a platform which easily lends itself to the use of computers, thereby allowing a diagnostic evaluation in minimal time. However, due both to the integration with respect to time of the ECG signals and the use of a statistical analysis which ignores the nature of the heart's electrical field, the isointegral maps still fail to provide a diagnosis in some instances. This occurs mainly because the discriminant function uses information values from specific electrode sites, which have been weighted in terms of their relevance to the disorder being investigated. Yet another BSM apparatus which utilizes the ST component of a heartbeat to perform analysis of the heart is described in U.S. Patent No. 5,419,337, and an electrode harness described in pending U.S. Application Serial No. 08/553,101, filed November 3, 1995.

By experience, it has been found that the 12 lead ECG is only around 50% sensitive in the detection of acute myocardial infarction (M1), as reported in "Initial Diagnosis of Myocardial Infarction: Body Surface Mapping" by S. Hameed, R. Patterson, J. Allen, S. McMechan, G. MacKenzie, J. Anderson and J. Adgey of Regional Medical Cardiology Centre, Royal Victoria Hospital, Belfast, Northern Ireland. The authors investigated a body surface mapping (BSM) system incorporating a 64 lead harness applied to the anterior chest in patients with suspected MI. At each of the 64 points QRS and ST-T integrals were measured. Features extracted from the subsequent isointegral maps were used to described the shape of the maps obtained. Thus, it was shown how through the use of BSM, higher sensitivity and specificity could be achieved for the discrimination of MI and non-MI patients with chest pain over that achievable using the standard 12 lead ECG.

Many techniques have been used in the past by cardiologist's attempting to identify the direction and significance of the hearts activation. Of particular note is the spatial VectorCardioGraphic ( VCG ) loop, "Heart ", Frank H Netter MD, The CIBA collection of medical illustrations, 1991, p52. In its standard form, this technique used six electrodes placed on the body so as to measure three orthogonal bipolar leads. The VCG loops presented by the technique were viewed as three orthogonal planes namely Frontal, Horizontal and Sagittal. The technique attempted to show how the heart activated as a single bipolar vector or dipole. The above reference also considers the well accepted concept of a mean cardiac vector or axis. This is used to provide a perceived impression of what the heart is doing electrically at a given instant of time. The mean cardiac vector would point in the direction in which the majority of the heart is activating. The vector also attempts to portray magnitude as an indication of how much myocardium is activating in the direction in which the vector is pointing. The above reference shows how similar the VCG and the mean cardiac vectors are for a normal healthy individual.

Clinicians attempting to make a diagnosis of a patient with chest pain using either the twelve lead ECG or the VCG have found them to be unhelpful in many instances. It is thought that this is mainly due to these devices not having electrodes on the body surface over the area of myocardium which is being aberrantly affected. As the heart activates, there are many tissues generating electrical signals in many directions at many instants throughout the cardiac cycle. The heart is also a hollow three dimensional organ divided primarily into two main chambers. The use of such single dipole techniques therefore could be concealing important electrophysiological information, regarding the origin of normal and/or abnormal activations from different regions of the heart. It is these complexities which mean that the true mean cardiac vector cannot be calculated using either VCG or currently available ECG apparatus. Using the current techniques, it is possible that an aberration of the posterior myocardium will present itself in the same direction and magnitude as an aberration of the anterior myocardium. Currently the only method of separating the two regions is to invasively explore the heart from within the thoracic cavity (epicardial mapping) or from within the heart chambers themselves (endocardial mapping). BSM overcomes the limitations of these systems by using a proliferation of electrodes over the entire thorax. As mentioned earlier however, the BSM method increases the complexity of the problem to the point where using the method requires a period of time which is not a practical in acute situations.

### Summary of The Invention

It is, therefore, an object of the present invention to provide a means by which activation from different regions of the heart may be detected non-invasively from the body surface, for example using body surface mapping apparatus disclosed in British Specification No. GB 2 264 176 (US No 5 419 337), and as such provide electrophysiological indications of aberrant cardiac activation.

Furthermore, it is an object of the present invention to provide a means of detecting abnormal changes in the electrophysiological activation of the heart in such a way so as to allow this activity to be used to diagnose the condition of an individual's myocardium and thereby allow their condition and subsequent treatment to be managed.

It is also an object of the present invention to allow this activation to be presented in a form which can either be viewed graphically for interpretation or analyzed using mathematical algorithms.

European Patent Specification No. EP-A-0 512 719 discloses a system that includes a monitor (16) for use in performing 12-lead electrocardiographic (ECG) and body surface mapping (BSM) analyses on a patient with a single ten-electrode cable set (14). The monitor receives nine leads of data from the electrode cable set and initially preprocesses it to simplify further analysis.

According to an aspect of the present invention, there is disclosed a method as specified in claim 1.

The invention will be understood in greater detail from the following description of a preferred embodiment thereof given by way of example only and with reference to the accompanying drawings in which:
Figure 1 depicts an isopotential map superimposed onto an outline of the human torso.
Figure 2 (top) depicts assigned free resultant vector components originating from the WCT.

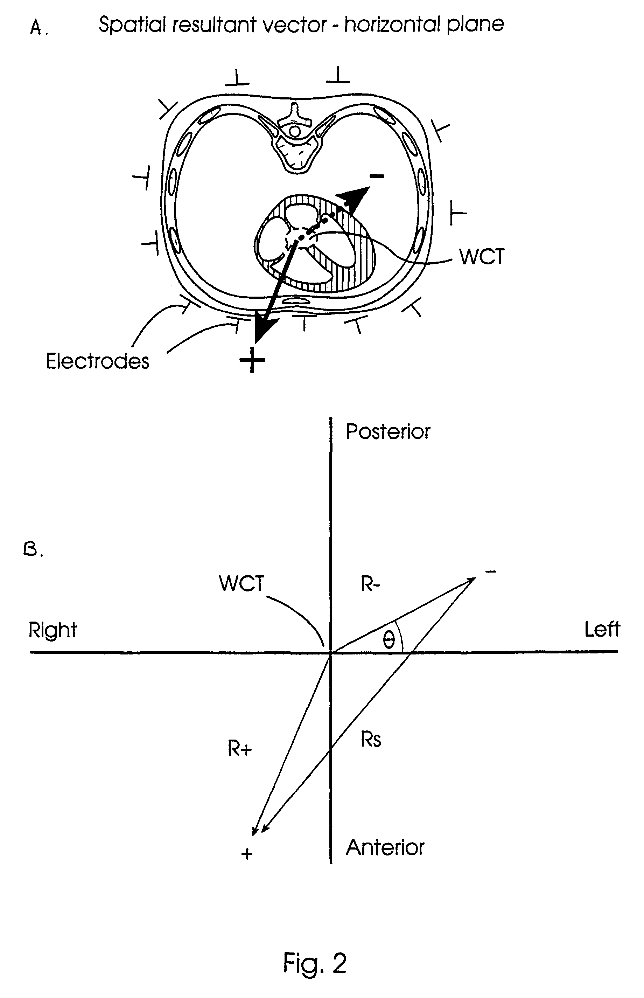

Figure 2 (bottom) is a vector plot of the vector components summed as a spatial resultant vector.

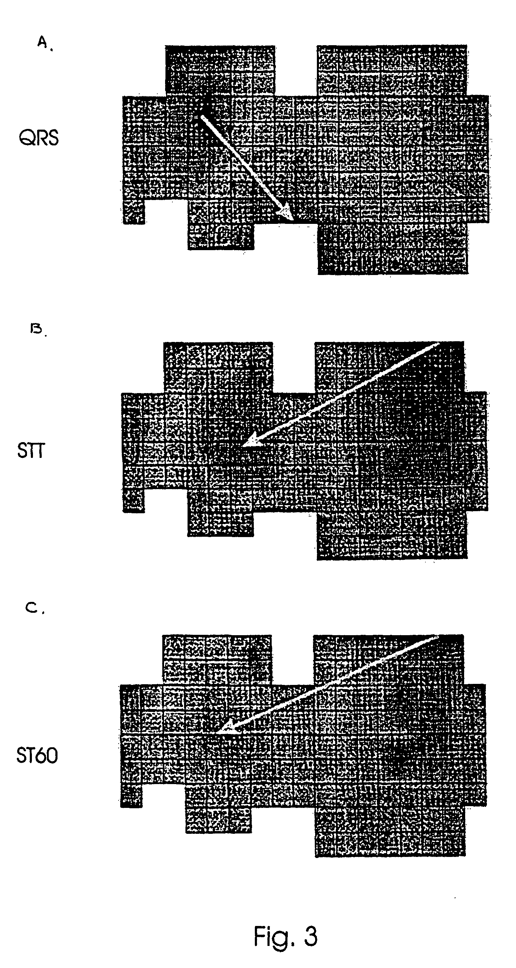

Figure 3 is a display showing spatial resultant vectors for a normal healthy control subject.

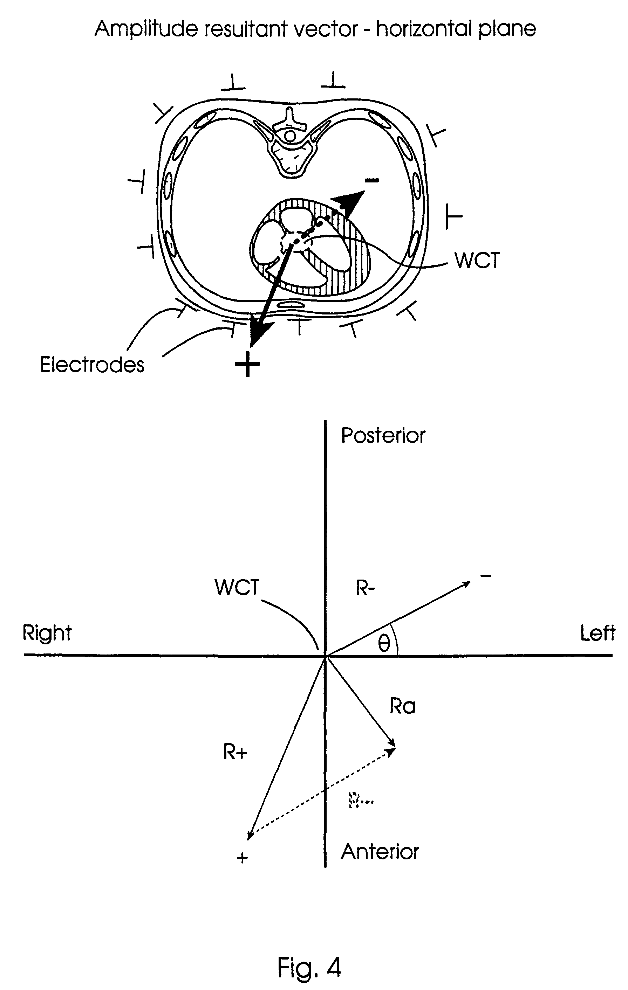

Figure 4 (top) depicts assigned free resultant vector components originating from the WCT.
Figure 4 (bottom) is a vector plot of the vector components summed as an amplitude resultant vector.

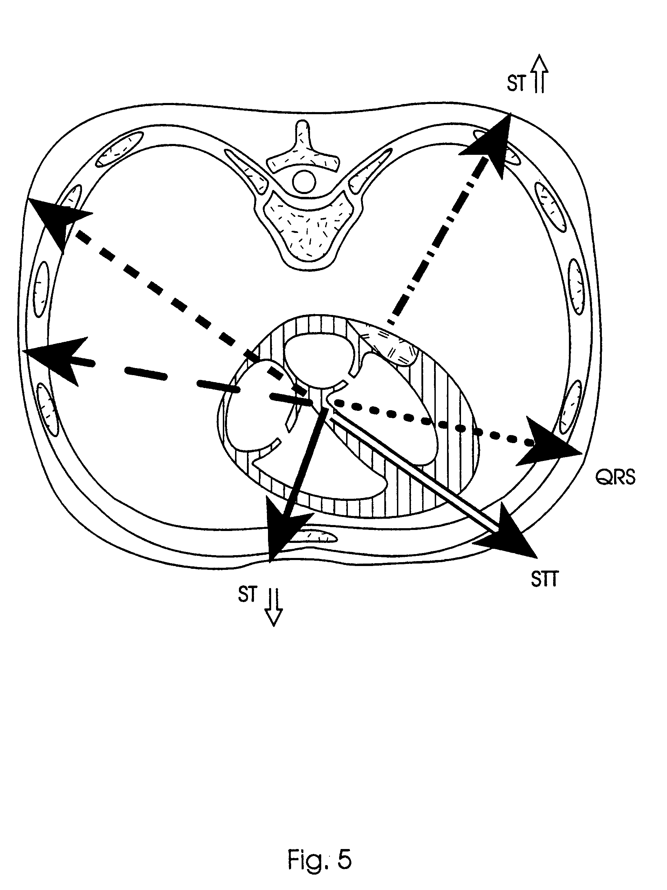

Figure 5 is a sketch of spatial resultant vectors for the two isointegrals QRS and STT, and a spatial resultant vector for the ST60 isopotential (for example only).

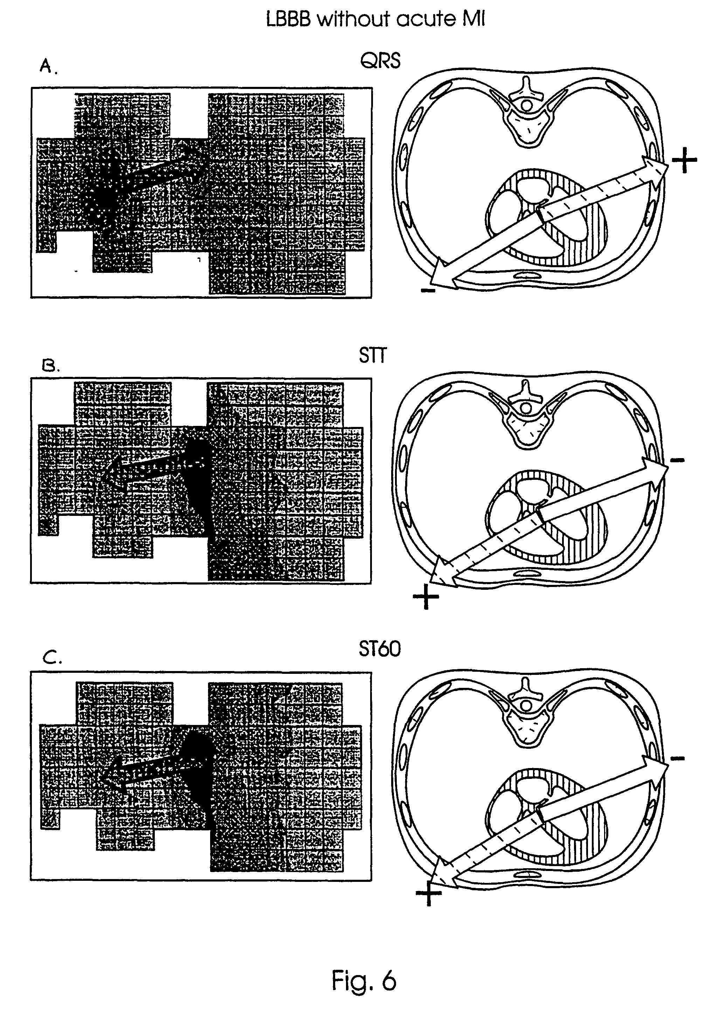

Figure 6 shows actual spatial resultant vectors for a patient suffering LBBB.

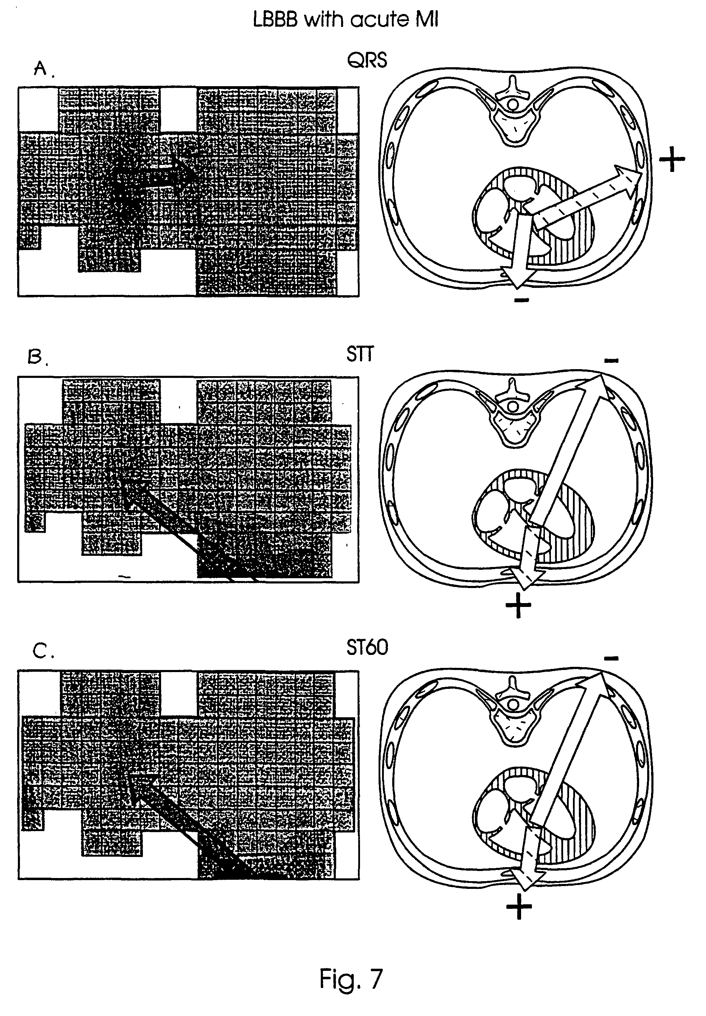

Figure 7 shows actual spatial resultant vectors for a patient suffering an acute MI complicated by LBBB.

The invention provides a plurality of free vector components which are referenced to a point located at the electrical centre of the heart (Figure 2). This electrical centre is well documented as the Wilson Central Terminal (WCT). These vector components are formed in pairs each one of which is individually given a size and direction with reference to the WCT origin and each is allowed complete freedom to explore three dimensional space. The size and direction are assigned according to pre-defined unipolar measurements obtained from a plurality of locations on the body surface. The body surface being considered as a three dimensional surface enclosing the human thorax.

As an example Figure 2 shows assigned resultant QRS isointegral vectors. The "+" and "-" signs in this instance denote the location of the maximum "+" and minimum "-" values which were measured across the entire body surface, which was mapped using body surface mapping apparatus (such as that referenced above) which recorded information from a total of 80 electrodes simultaneously. In this figure the vectors are shown as arrows, however any symbol denoting magnitude and direction could be used for this purpose without departing from the scope of the invention. Note how each individual component vector possesses angular direction and a size in terms of it's integral value at the given location on the body surface. In addition to the assigned resultant QRS vectors, the plot at the bottom of Figure 2 shows the difference of these two vectors calculating the spatial resultant vector (or spatial cardiac axis) "Rs" which was described in the background to the invention above. The figure shows just one of three possible views namely the horizontal plane. Figure 3 shows this calculation being performed for the QRS (from onset of QRS to J point) interval, STT (from J point to end of T wave) interval and the ST60 (instantaneous measurement 60 milliseconds into the ST segment from the J point) instant of a healthy control subject. The invention has correctly located the spatial cardiac vector using 80 unipolar electrodes in three dimensional space. The bipolar systems previously described cannot achieve this precision since they rely upon orthogonal redundancy assuming a single cardiac dipole type activation. The invention also allows calculation of the amplitude resultant vector. Figure 4 shows the construction of this vector for the same example. Here the pair of free resultant component vectors are summed in the vector plot to identify the overall amplitude asymmetry generated by the heart. Note that any period or instant referenced from any time instant could be employed without departing from the scope of the invention.

It can now be appreciated that both the resultant spatial vectors and resultant amplitude vectors use information obtained from the free resultant component vectors on electrically opposite sides of the WCT.

The use of the invention involves examination of the direction and amplitude of the resultant component vectors and vector measurements made from them. Figure 5 shows two vectors namely QRS isointegral and STT isointegral for a normal healthy individual. Notice these resultant vectors span opposite regions of the thorax and are thereby are not required to be individually free. If one now introduces say an ischaemic injury current into the heart's electrical field (shaded region within free wall of left ventricle), one would expect the STT vector to change amplitude and or direction. The following example will demonstrate how using the WCT as a reference for unipolar resultant component vectors and allowing said vectors to have individual angular freedom in three dimensional space can reveal changes due to (for example) ischaemic injury.

Let us now consider the diagnosis of acute MI complicated by LBBB (a challenge to all electrocardiographic diagnostic systems). The three resultant plots of Figure 6 show what has been termed complete resultant cardiac reversal. The plots on the right of the figure show the resultant vectors assigned to the maximum and minimum values across the body surface for each of the three measurements QRS, STT and ST60. The plots to the left show the calculated spatial resultant vectors for each respective measurement. The assigned component vectors experience a complete polarity reversal from the QRS to both the STT and ST60 plots. The calculated spatial resultant vector also show a complete STT and ST60 reversal from the vector shown in the QRS. This effect can be explained in terms of cellular activation. Briefly, the presence of a LBBB has slowed ventricular depolarisation and caused repolarisation to occur in the opposite direction than would occur in a normal healthy heart.

Figure 7 now shows the same plots for a patient suffering an acute MI complicated by LBBB. Here, however, the assigned vectors contain a distinct angular shift which is presented due to the injury current also present within the heart's electrical field. This shift is also correctly calculated and presented in the respective spatial resultant vectors. The injury current has been identified and therefore denotes acute MI.

It is now possible to accurately measure these directions, angular shifts and amplitude changes to characterize the condition of subjects with known disorders in addition to detecting, comparing and diagnosing many different disorders of presented patients. The information once obtained can either be graphically presented (as done so here) or used as the input to a discriminatory algorithm, using computer programming techniques known to those skilled in the art. This will aid the operator in determining treatment and/or medication for that individual.

The invention has also been used to identify injury currents within cardiac activity of hearts which are not suffering cardiac arrhythmias.

The apparatus used for the diagnostic technique described above may be based upon that disclosed in British Specification No. GB 2 264 176 (US No 5 419 337). Such apparatus comprises a plurality of electrodes which are in use attached to spatially separate locations on a human torso, each electrode being capable of detecting the electrical activity associated with a heartbeat and producing a corresponding signal. The apparatus is further programmed, in carrying out the present invention, to process the signals from the electrodes to calculate and present (graphically or otherwise) to the user the desired QRS, ST-T and ST60 map vectors and related cardiac vectors. Alternatively, as stated above, the calculated values of the QRS, ST-T and ST60 map vectors can be used as the input to a discriminatory algorithm. Details of such apparatus, apart from the programming which is well within the capabilities of those skilled in the art, can be obtained from the aforesaid British Specification No. GB 2 264 176 (US No 5 419 337).

## Claims

1. A method of using body surface mapping to determine the condition of a human heart, the method comprising attaching a plurality f electrodes to spatially separate locations on a human torso, each electrode being capable of detecting the electrical activity associated with a heartbeat and producing a corresponding signal, **characterised in that** the electrodes extend substantially fully around the torso, and **in that** the signals are processed to calculate QRS, ST-T and ST60 vector components substantially in a plane passing through the Wilson Central Terminal (WCT), each vector component representing the electrical activity of the heart with respect to the WCT.

2. A method as claimed in claim 1, further including calculating the QRS, ST-T and ST60 spatial resultant vectors.

## Patentansprüche

1. Verfahren zur Verwendung des Körperoberflächen-Mapping zur Bestimmung des Zustandes eines menschlichen Herzens, wobei das Verfahren das Aufbringen einer Vielzahl von Elektroden an räumlich getrennten Stellen auf einem menschlichen Torso umfasst, wobei jede Elektrode zum Nachweis der mit einem Herzschlag einhergehenden elektrischen Aktivität fähig ist und ein entsprechendes Signal produziert, **dadurch gekennzeichnet, dass** die Elektroden weitgehend vollständig um den Torso herum verlaufen und dass die Signale zur Berechnung der QRS-, ST-T- und ST60-Vektorkomponenten, die weitgehend in einer Ebene durch das Wilson Central Terminal (WCT) passieren, wobei jede Vektorkomponente die elektrische Aktivität des Herzens in Bezug auf das WCT darstellt, verarbeitet werden.

2. Verfahren nach Anspruch 1, weiter einschließend die Berechnung der resultierenden Raumvektoren von QRS-, ST-T- und ST60.

## Revendications

1. Procédé d'utilisation de la cartographie de la surface corporelle pour déterminer la condition d'un coeur humain, le procédé comprenant la fixation d'une pluralité d'électrodes à des emplacements spatialement séparés sur un torse humain, chaque électrode étant capable de détecter l'activité électrique associée à un battement cardiaque et produisant un signal correspondant, **caractérisé en ce que** les électrodes s'étendent sensiblement totalement autour du torse, et **en ce que** les signaux sont traités pour calculer les composants de vecteurs QRS, ST-T et ST60 sensiblement dans un plan passant à travers le point de Wilson, chaque composant de vecteur représentant l'activité électrique du coeur par rapport au point de Wilson.

2. Procédé selon la revendication 1, comprenant en outre le calcul des vecteurs spatiaux résultants QRS, ST-T et ST60.
